# EUROPEAN PATENT APPLICATION

(11) **EP 0 569 086 A2**
(43) Date of publication of application: **10.11.1993**
(21) Application number: 93201243.8
(22) Date of filing: 01.05.1993
(51) Int. Cl.: G01N 33/547, G01N 33/545, G01N 33/68, C12Q 1/68

(54) **Method and kit for attaching compounds to carboxylated substrates using uronium salt**

(30) Priority: 06.05.1992 US 879239
(71) Applicant: Johnson & Johnson Clinical Diagnostics, Inc., Rochester New York 14650 (US)
(72) Inventor: Danielson, Susan J., c/o Eastman Kodak Co., Rochester, NY 14650-2201 (US); Specht, Donald P., c/o Eastman Kodak Co., Rochester, NY 14650-2201 (US)
(74) Representative: Mercer, Christopher Paul

(57) **Abstract**

Certain uronium salts are useful for attaching biological or chemical compounds having a reactive amino or sulfhydryl group to a carboxylated substrate, such as carboxylated particles. These salts are 2-(N-heterocyclyloxy)uronium salts which activate reactive carboxy groups for reaction with the amino or sulfhydryl groups. The resulting reagents are useful in various analytical, diagnostic and medical applications, or can be used as conjugates in various immunoassays.

## Description

This invention relates to a method for the preparation of reagents from carboxylated substrates, and particularly water-insoluble substrates, using a uronium salt activating agent. It also relates to a kit useful in the practice of this method. This invention is particularly useful in the field of diagnostic chemistry.

Biologically active proteins or other molecules have been attached to water-insoluble carriers for some time. The resulting reagents are useful in the diagnosis of pathological or other conditions (such as pregnancy) in humans and animals using immunological principles for the detection of a target immunological species indicative of the condition. In most instances, the target immunological species is an antigen or antibody specific for an antigenic material.

Other biological compounds having reactive sulfhydryl or amino groups have been attached to polymeric particles or other substrates for use in affinity chromatography, enzymatic reactions, purification procedures, nucleic acid capture and specific binding assays as well as immunoassays. Magnetic particles, latex particles, erythrocytes, bacterial cells and other particulate materials have been used as carrier materials in such technologies.

Carboxylated latex particles have been used to prepare diagnostic reagents as described, for example, in US-A-4,181,636. The most common method for attaching an antibody to such particles involves the use of a water-soluble carbodiimide. While producing useful reagents, this procedure tends to activate exposed reactive groups of the antibody as well as the carboxyl groups on the particles. The result is intramolecular and intermolecular crosslinking or polymerization of the antibody. A significant portion of the antibody is thus impaired from complexation with a corresponding antigen. Because the antibody (and many other biological species) is relatively costly, this problem represents a serious economic loss. The use of carbodiimides is inefficient compared to more recently discovered procedures.

EP-A-0 308 235 describes the use of carbamoylonium compounds to attach biologically active compounds to carboxylated particles. While this procedure provides considerable improvement in efficiency over the use of carbodiimides, there was a need for further improvement because, in some instances, the carbamoylonium compounds generate strongly nucleophilic amine by-products which compete with the compounds to be attached for active sites on the particles.

Additional improvements over the use of carbodiimides is provided by using a dication ether, as described in EP-A-0 411,711.

More recently, it was found that certain 1-(1-pyrrolidinylcarbonyl)pyridinium salts avoid the formation of amine by-products encountered with the carbamoylonium compounds, and they provide very high levels of covalent attachment of the desired compound to the particles with high retention of activity by the attached compounds (such as antibodies). However, such pyridinium salts are not commercially available, and the procedures for making them on a commercial scale may present a problem. The salts are hygroscopic and must be kept free of moisture which creates some manufacturing difficulties. Designing effective manufacturing processes to keep the products dry at all times would increase production costs.

In the highly competitive diagnostics market, there is an acute need to reduce the costs of reagents so the resulting methods and test kits are economically competitive. However, there is no desire to obtain such economic advantages by giving up either high efficiency for attachment of compounds to carboxylated particles or reagent stability.

The problems noted above are overcome with the use of a kit comprising a water-insoluble substrate having reactive carboxy groups,
the kit characterized as further comprising, individually packaged, a 2-(N-heterocyclyloxy)uronium salt represented by the structure: 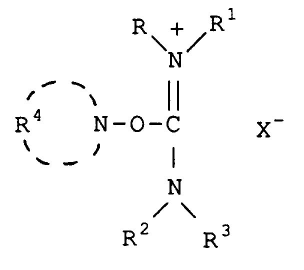
wherein R, R¹, R² and R³ are independently hydrogen, substituted or unsubstituted alkyl having 1 to 6 carbon atoms, substituted or unsubstituted alkenyl having 2 to 6 carbon atoms, or alkynyl having 2 to 6 carbon atoms, or
R and R¹, when taken together, or R² and R³, when taken together, represent the carbon, sulfur, nitrogen and oxygen atoms necessary to complete a 5- to 7-membered substituted or unsubstituted heterocyclic ring with the nitrogen atom to which they are attached, or
R¹ and R³, when taken together, represent the carbon, sulfur, nitrogen and oxygen atoms necessary to complete a 5- to 7-membered substituted or unsubstituted heterocyclic ring with the uronium group to which they are attached,
R⁴ represents the carbon and heteroatoms which, when taken with the nitrogen atom attached to the oxy group, are sufficient to complete a 5- to 10-membered substituted or unsubstituted heterocyclic ring, and
X⁻ represents an acid anion.

This invention also provides a method for reacting a biologically active compound having a reactive amino or sulfhydryl group with a water-insoluble carboxylated substrate comprising:
A. contacting a water-insoluble substrate having reactive carboxy groups with a 2-(N-heterocyclyloxy)uronium salt, as described above, to convert the reactive carboxy groups to activated groups in the substrate, and
B. contacting the substrate having activated groups with a biologically active compound having a reactive amino or sulfhydryl group which reacts with the activated groups to form a covalent linkage between the substrate and the biologically active compound.

The present invention provides a means for rapid and highly efficient attachment of reactive amine- or sulfhydryl-containing compounds to carboxylated substrates, such as carboxylated particles or drugs having reactive carboxy groups. This attachment is achieved using a certain class of uronium salts described in detail below. High efficiency of attachment and reagent stability are achieved, but in addition, the activating agents described herein are readily available so that economic advantages are also obtained. In particular, the preferred compound, 2-(1H-benzotriazol-1-yloxy)-1,1,3,3-tetramethyluronium tetrafluoroborate, is commercially available.

The reagents prepared according to the method of this invention can be used in many different chemical and biological procedures. For example, they can be used in affinity chromatography, reactions catalyzed by enzymes, water purification, immunoassays and other processes which would be readily apparent to one skilled in the art. In some instances, the present invention can be used to attach intermediate linking substituents which can be further reacted with compounds of biological or chemical interest.

The biologically active compounds or linking substituents which can be attached to carboxylated substrates have at least one reactive amino or sulfhydryl group. The term "biologically active" is meant to refer to compounds or molecules which have a capacity to interact with another component which can be found in a physiological fluid. Such interaction can be catalytic activity as in the case of an enzyme. Alternatively, it can be a specific binding interaction such as between antibodies and antigens, sugar and lectins or avidin and biotin. Such biologically active compounds include, but are not limited to, nucleotides, oligonucleotides, nucleic acids, amino acids, peptides, polypeptides, proteins (including lipoproteins and glycoproteins), modified carbohydrates (including modified polysaccharides), hormones, drugs, steroids (other than those included in previous categories), vitamins, glycolipids, viruses or components thereof, microorganisms or components thereof, fungi or components thereof, molds or components thereof, blood components, organ components, lectins and other compounds which would be readily apparent to one skilled in the art. Preferably, the attached compounds are polypeptides, proteins, derivatives of carbohydrates, haptens, hormones, lectins, vitamins (such as biotin) oligonucleotides and nucleic acids. More preferably, they are proteins (including antibodies) or oligonucleotides.

For example, the attached compound can be an antibody specific to any of a wide range of antigenic materials including protein or carbohydrate components of microorganisms, drugs or various hormones. Representative antibodies are those specific to thyroxine, carbamazepine, dilantin, phenobarbital, phenytoin, C-reactive protein, human chorionic gonadotropin, Streptococcus A or B, a microorganism associated with periodontal diseases (such as *Actinobacillus actinomycetemcomitans, Prevotella intermedia and Prophyromonas gingivalis*), herpes, rubella, hepatitis and other viruses (including retroviruses such as HIV-I).

The attached compound can be an antigenic component of a virus or microorganism which is then used to complex with an antibody of interest, such as a viral antibody.

In still another embodiment, the attached compound can be an oligonucleotide which has been modified to have the requisite amino or sulfhydryl group for attachment. Procedures for modifying oligonucleotides are described, for example, in US-A-4,914,210 and WO-89/2932, US-A-4,719,182, Erlander and others, J.Biol.Chem., 234, 1090 (1959), Wiston and others Biochem.Biophys.Acta, 612, pp. 40-49 (1980) and Borzini and others, J.Immunol.Methods, 44, pp. 323-332 (1981).

In still another embodiment, the method of this invention can be used to attach or conjugate proteins, amino acids or oligonucleotides to drugs having reactive carboxy groups. For example, enzymes can be conjugated with drugs or haptens in this manner, for use in immunoassays. Other applications would be readily apparent to one of ordinary skill in the art.

In a preferred embodiment, the substrates are water-insoluble, such as in the case of water-insoluble particles having surface carboxy groups. The resulting reagents (for example, compounds attached to particles) can be used in various immunoassay formats, including but not limited to agglutination assays, solution and dry competitive immunoassays, enzyme-linked immunosorbent assays (ELISA), radioimmunoassays (RIA) and immunometric (or sandwich) assays. Dry competitive immunoassays are generally carried out using dry analytical elements. The details of useful assay formats and reagents are not provided here because they are well known in the art, as described for example in EP-A-0 462 644.

The method of this invention has a minimum of two steps: (1) activating the carboxy groups in the substrate to produce activated groups in the substrate, and (2) reacting the activated groups with reactive amino or sulfhydryl groups in the compound to be attached.

As used herein in the preferred embodiment, a water-insoluble substrate includes any carboxylated material in any suitable shape or form to which biologically active compound be attached. The substrate can be composed of a suitable polymer, glass, ceramic, metal, magnetic composite or mixtures of the foregoing. Such substrates can be in the form of cuvettes, films, test tubes, microtiter plates, membranes and other shaped materials which have carboxy groups on the surfaces thereof. The carboxy groups can be provided by preparing the substrate from monomers or polymers having carboxy groups, or they can be provided by further chemical treatment of a non-carboxylated surface after the substrate is formed.

Preferably, the carboxylated substrate is a carboxylated particle, and the remainder of this discussion will be directed to such particles for exemplification purposes. As used herein, "carboxylated particles" refers to water-insoluble particles formed of glass, ceramics, naturally occurring or synthetic polymers which have sufficient carboxy groups on the surface for attachment of sufficient molecules for a particular use. Preferably, the particles are polymeric latex particles generated by emulsion or suspension polymerization (including batch, continuous or semi-continuous techniques). It is preferred that the particles be entirely free of surfactants or colloidal suspension agents when the biological compounds are attached. This can be generally achieved by lengthy periods of dialysis or other purification of the polymeric suspensions or latices. However, it is most preferred that the polymeric particles be prepared without the use of surfactants or colloidal suspension agents because then there is assurance that none is available during the attachment method. Methods for preparing surfactant-free latices are described in US-A-4,415,700 and Research Disclosure publication 15963 (July, 1977).

The water-insoluble particles generally have a particle size in the range of from 0.01 to 100 µm, and preferably in the range of from 0.1 to 10 µm. They can be homogenous particles meaning that they are composed of the same material throughout, or they can be composites, core-shell particles or coated or grafted with different materials as is known in the art (for example, US-A-3,700,609, US-A-4,401,765 and US-A-4,997,772).

The particles have surface carboxy groups which can be provided in a number of conventional ways. Glass particles can be treated, for example with organosiloxanes having carboxyalkyl groups (similar to the conventional organosiloxanes used to make sol gel glasses) to provide surface carboxy groups. Core-shell and graft polymer particles can be provided as known in the art using monomers which provide the reactive carboxyl groups. Alternatively, particles can be prepared from monomers having groups which, upon further reaction can be converted to carboxy groups (for example, hydrolysis of anhydrides or oxidation of methylol or aldehyde groups). More preferably, the particles are homogenously prepared from one or more monomers containing pendant carboxy groups, many of which monomers are well known in the art.

Useful carboxylated particles can be prepared from homopolymers of carboxylated styrene and its derivatives, carboxylated styrene-butadiene copolymers, acrylic and methacrylic acid, itaconic acid and other ethylenically unsaturated carboxylic acids and equivalent salts and anhydrides.

Preferably, the particles are prepared from polymers derived by addition polymerization from:
a. 0.1 to 100 mole percent of an ethylenically unsaturated polymerizable monomer having a carboxy group (or equivalent salts or anhydride precursors), and
b. 0 to 99.9 mole percent of an ethylenically unsaturated polymerizable monomer not having a carboxy group.

Monomers from which component (a) can be derived include, but are not limited to, acrylic acid, methacrylic acid, itaconic acid, aconitic acid, fumaric acid, maleic acid, β-carboxyethyl acrylate, β-carboxyethyl methacrylate, m & p-carboxymethylstyrene, methacrylamidohexanoic acid and N-(2-carboxy-1,1-dimethylethyl)acrylamide or a salt or anhydride precursor thereof.

Monomers from which component (b) can be derived are those which when homopolymerized, provide a water-insoluble, hydrophobic polymer, and include, but are not limited to vinyl aromatics (both monovinyl and divinyl compounds, such as styrene, vinyltoluene, 4-t-butylstyrene, 2-chloromethylstyrene and divinyl-benzene), acrylic and methacrylic acid esters and amides (such as methyl acrylate, ethyl acrylate, n-butyl acrylate, 2-ethylhexyl methacrylate, methyl meth-acrylate, 2-hydroxyethyl methacrylate, methacrylamide, ethylene dimethacrylate and 2-hydroxyethyl acrylate), p-styrenesulfonate, acrylonitrile, sodium 2-acrylamido-2 methylpropanesulfonate, sodium 3-acryloyloxypropanesulfonate and others readily apparent to one skilled in the art. Preferably, component (b) is derived from one or more vinyl aromatic monomers.

Preferably, the copolymer defined above has from 1 to about 20 mole percent of component (a) and from 80 to 99 mole percent of component (b).

Particularly useful carboxyl-containing monomers are described in detail in EP-A-0 466 220. These monomers are generally described by the structure:

CH₂ = CR'-L-COOM

wherein R' is hydrogen, halo or alkyl of 1 to 3 carbon atoms, M is hydrogen, ammonium ion or an alkali metal ion, and L is a divalent organic linking group having from 8 to 50 carbon, nitrogen, oxygen or sulfur atoms in the linking chain. Representative monomers are described in the noted reference. The monomer 3-(p-vinylbenzylthio)propionic acid is one of the preferred monomers in this class.

Representative polymers useful in the practice of this invention include, but are not limited to: poly (styrene-co-acrylic acid), poly(styrene-co-vinylbenzyl chloride-co-methacrylic acid), poly-(styrene-co-acrylic acid-co-divinylbenzene), poly-(methyl methacrylate-co-itaconic acid), poly(styrene-co-butyl acrylate-co-acrylic acid), poly (styrene-co-mono-2-methacryloyloxyethyl glutarate), poly[styrene-co-monomethacryloyldeca(oxyethylene)glutarate], poly[styrene-co-2-(m & p-vinylbenzylthio)ethyl glutarate], poly[styrene-co-2-(4-vinylbenzylthio)-benzoic acid], poly{{styrene-co-mono-2-{2-[2-(4-vinylbenzylthio)ethoxy]-ethylthio}ethyl phthalate}}, poly{styrene-co-mono-methyacyloyldeca(oxyethylene)phthalate-co-mono-deca(oxyethylene)methacylate] and poly[styrene-co-3-p-(vinylbenzylthio)propionic acid], all in various monomer ratios.

The carboxylated particles described herein can be supplied as a dry powder which can be suspended in an aqueous solution for use in the invention. Preferably, they are supplied as a suspension at from 0.1 to 35 percent solids. Buffers, preservatives or other addenda can be included if desired.

A specific class of uronium salts are useful in this invention to activate the carboxylated substrate (for example, carboxylated particles) so the biologically active compounds of interest can be covalently attached thereto. These compounds are represented by the structure: 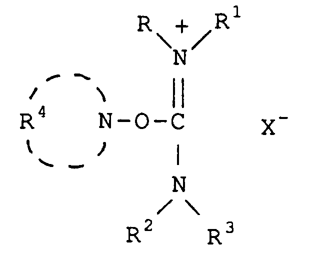
wherein R, R¹, R² and R³ are independently hydrogen, substituted or unsubstituted alkyl having 1 to 6 carbon atoms (such as methyl, ethyl, isopropyl, t-butyl, hexyl, chloromethyl and methoxymethyl), substituted or unsubstituted alkenyl having 2 to 6 carbon atoms (such as ethenyl, allyl, 1-propen-2-yl 2-hexen-1-yl, 3-methyl-1-penten-1-yl), or alkynyl having 2 to 6 carbon atoms (such as 2-propyn-1-yl, 2-hexyn-1-yl or 2-ethyl-3-butyn-1-yl), or
R and R¹, when taken together, or R² and R³, when taken together, represent the carbon, sulfur, nitrogen and oxygen atoms necessary to complete a 5- to 7-membered substituted or unsubstituted heterocyclic ring with the nitrogen atom to which they are attached (for example, piperidine or piperidinium, pyrrolidine or pyrrolidinium, morpholine or morpholinium, piperazine or piperazinium, 4-methylpiperazine or 4-methylpiperazinium, and hexahydroazepine or hexahydroazepinium rings). The rings on the molecule can be the same or different.

R¹ and R³, when taken together, can also represent the carbon, sulfur, nitrogen and oxygen atoms necessary to complete a 5- to 7-membered substituted or unsubstituted heterocyclic ring with the uronium group to which they are attached (for example, imidazolium, pyrimidinium, triazinium and azepinium rings).

Preferably, R, R¹, R² and R³ are the same monovalent group, or R and R¹ taken together form the same heterocyclic ring as R² and R³ taken together so the molecule is symmetrical. More preferably, R, R¹, R² and R³ are each hydrogen alkyl of 1 to 3 carbon atoms or alkenyl of 2 to 4 carbon atoms, and most preferably, each is methyl.

The heterocyclic rings described above can be substituted with one or more substituents at any position which do not adversely affect the compound's ability to activate carboxy groups in the method of this invention. For example, the rings can be substituted with one or more alkyl groups having up to 6 carbon atoms (for example, methyl, ethyl, isopropyl, hexyl and 2-methoxypropyl), halo (for example, chloro or bromo) and alkoxy having up to 6 carbon atoms (for example, methoxy, propoxy, isobutoxy, 2-methoxypropoxy and hexoxy). Preferred substituents include one or two lower alkyl groups such as methyl and ethyl. More preferably, the heterocyclic rings described above are unsubstituted and saturated.

In the foregoing structure, R⁴ represents the carbon and heteroatoms (for example, sulfur, oxygen or nitrogen) which, when taken with the nitrogen atom attached to the oxy group, are sufficient to complete a 5- to 10-membered heterocyclic ring. Such heterocyclic rings are substituted or unsubstituted and include, but are not limited to, 1-benzotriazolyl, succinimido, 1-imidasolyl, 2-imidazolidinon-1-yl, morphlino, pyridyl, thiazolyl, benzothiazolyl, quinolyl, piperidyl, piperazinyl, pyrrolyl, pyrazinyl, pyrimidyl and pyridizinyl. Such rings can be substituted with one or more lower alkyl (methyl, ethyl, isopropyl or butyl), oxo, halo (for example, chloro), lower alkoxy (such as methoxy, propoxy, isobutoxy and ethoxy). Preferably, the ring is unsubstituted or has up to two of such substituents (the same or different). Also preferably, the heterocyclic ring has from 5 to 9 atoms in the ring. More preferably, the ring is 1-benzotriazolyl or succinimido, with 1-benzotriazolyl being most preferred.

Also in the structure noted above, X⁻ represents an anion or an anionic portion of the compound needed to form an intramolecular salt. Representative anions include, but are not limited to, halide (such as chloride, bromide or fluoride), tetrafluoroborate, triflate, nitrate, sulfate, p-toluenesulfonate, perchlorate, methosulfate, hydroxide and hexafluorophosphate. X⁻ can also be a sulfonate or an alkylenesulfonate attached to the heterocyclic ring, the alkylene portion having from 1 to 6 carbon atoms. The tetrafluoroborate salts are preferred in the practice of this invention.

Representative activating agents useful in the practice of this invention include, but are not limited to, 2-(1H-benzotriazol-1-yloxy)-1,1,3,3-tetramethyluronium tetrafluoroborate, 2-(succinimid-oxy)-1,1,3,3-tetramethyluronium tetrafluoroborate, 2-(3,4-dihydro-4-oxo-1,2,3-benzotriazin-3-yl)-1,1,3,3-tetramethyluronium tetrafluoroborate, 2-[2-(1H)-pyridon-1-yl]-1,1,3,3-tetramethyluronium tetrafluoroborate, 2-(5-norbornene-2,3-dicarboximido)-1,1,3,3-tetramethyluronium tetrafluoroborate, 2-(2-pyrazolin-5-on-1-yl)-1,1,3,3-tetraethyluronium chloride, 2-(2-imidazolidinon-1-yl)-1,1,3,3-tetra-butyluronium p-toluenesulfonate, 2-(3-benzothiazolyl)-1,1,3,3-tetramethyluronium tetrafluoroborate, 2-[2-(1H)-quinolon-1-yl]-1,1,3,3-tetramethyluronium hexafluorophosphate, 1-[(1H-benzotriazol-1-yloxy)(1-pyrrolidinyl)methylene]pyrrolidinium tetrafluoroborate, 1-[1-piperidyl)(succinimidoxy)methylene]piperidinium triflate, 4-[(morpholino)(1-pyrrolidinyloxy)methylene]morpholinium chloride, 1-[(4-methyl-1-piperazinyl)(4-methyl-1-piperazinyloxy)methylene]-4-methylpiperazinium bromide and 1-[(N,N-dimethylamino)(succinimidoxy)methylene]pyrrolidinium hexafluorophosphate.

The first two activating agents listed above can be obtained commercially from Fluka Chemical Corporation (located in Ronkonkoma, New York). Others can be readily prepared using the teaching provided by Knorr and others, Tetrahedron Letters, 30, pages 1927-1930 (1989) and references noted therein.

In certain embodiments, the reagents prepared by this invention can have a tracer associated therewith. A tracer is a detectable species which enables one to detect the reagent. Useful tracers include, but are not limited to, dyes, radioisotopes and enzymes. Particularly useful tracers are colored or fluorescent dyes. The tracer can be associated with the reagent in any suitable fashion. For example, it can be associated (for example, covalently attached or ionically bound) with the attached biologically active compound (such as a radio-labeled protein or oligonucleotide). Alternatively, it is attached to the substrate, such as on the surface of, or within a water-insoluble particle. It is particularly desirable to incorporate fluorescent chelates or colored dyes into the particles using known techniques (for example, US-A-4,199,363, US-A-4,283,382 and US-A-4,997,772).

In the first step of the invention, the carboxylated particles are activated by the uronium salt activating agent at a suitable pH (from 3 to 7) and temperature (generally from 10 to 60°C). When the carboxylated substrate is a particle, the percent solids of the particles used in the method is generally from 0.01 to 30% with from 0.1 to 10% being preferred. The uronium salt is generally used at a molar ratio to the total measured carboxylic acid level on the substrate of from 1:1 to 200:1, and preferably from 10:1 to 100:1. This step may require up to 20 minutes.

The activated substrate (for example, particles) is then contacted (for example, by mixing) with the biologically active compound. When particles are used as the substrate, the activated particles are generally present at from 0.01 to 30% solids, and the biologically active compound is generally present in an amount of from 1:1000 to 2:1 weight ratio of compound to the substrate. This step is carried out at a pH in the range of from 3 to 8, and a temperature of from 10 to 50°C for up to 24 hours.

It may be desirable to remove excess activating agent once the reagents are prepared. Removal can be achieved by centrifugation or filtering the particles, accompanied by suitable washings and resuspensions in suitable buffers.

Further details of the method of this invention are provided in the representative examples below.

The carboxylated particles and uronium salt activating agent can be provided, in separate packaging, in a kit. Other materials, equipment and reagents which may be helpful can be included if desired. For example, the kit can also include a biologically active compound (such as an antibody) to be attached.

In the following examples, all percentages are by weight unless otherwise noted.

### EXAMPLES 1 & 2 Preparation of Reagent Having Bovine Gamma Globulin Attached to Polymeric Particles

These examples demonstrate the method of this invention for preparing a reagent having the protein bovine gamma globulin attached to carboxylated polymeric particles using two activating agents within the scope of this invention. The protein was radiolabeled for detection. A "Comparison" reagent was similarly prepared using an activating agent that is outside the scope of this invention.

Particles were prepared from two different polymers using the procedures described in EP-A-0 466 220 (noted above):
Polymer A: poly[styrene-co-3-(p-vinylbenzylthio)propionic acid] (97.59:2.41 molar ratio, 1.33 µm average diameter), and
Polymer B: poly[styrene-co-monomethacryloylpenta(oxyethylene) phthalate] (98.81:1.19 molar ratio, 1.3 µm average diameter).

The activating agents used in attaching the protein were:
Example 1: 2-(1H-benzotriazol-1-yloxy)-1,1,3,3-tetramethyluronium tetrafluoroborate,
Example 2: 2-(succinimidoxy)-1,1,3,3-tetramethyluronium tetrafluoroborate, and
Comparison: 1-(1-pyrrolidinylcarbonyl)pyridinium chloride.

The final suspensions of Polymer A particles and protein comprised 0.134 mg of ³H bovine gamma globulin per 30 mg dry weight of particles in 2-(4-morpholino)ethanesulfonic acid buffer (0.1 molar, pH 5.5). The final suspensions of Polymer B particles and protein comprised 0.164 mg of ³H bovine gamma globulin per 30 mg dry weight of particles in the same buffer. The amount of activating agent used in each experiment was 45 µmol.

The activating agent used in Example 1 (144.5 mg) was dissolved in buffer (4 ml, 112.5 mmolar stock solution). The activating agent used in Example 2 (135.5 mg) was dissolved in the same buffer (4 ml, 112.5 mmolar stock solution). The Comparison activating agent (95.9 mg) was dissolved in the buffer also (3 ml, 150 mmolar stock solution). Thus, to the suspensions described below, 400 µl of each of the activating agent solutions were added for Examples 1 and 2, and 300 µl of the Comparison activating agent solution was used in the Comparison method.

The reagent suspensions were prepared by adding the particles (30 mg dry weight) to a large microfuge tube, followed by addition of buffer to 1.5 ml. The resulting suspensions were centrifuged for 15 minutes at 14,000 rpm, and the supernatant was discarded. Buffer was added to each tube to provide a final volume of 1 ml, followed by addition of the activating agent (amounts noted above).

The tubes were then capped and rotated end-over-end for 10 minutes at room temperature to activate the carboxy groups on the particles. A solution of the radio-labeled protein (10 mg/ml, 13.4 µl for reactions with Polymer A and 16.4 µl for reactions with Polymer B) was added to each tube followed by addition of buffer to give a final volume of 1.5 ml. The tubes were again capped and rotated for 24 hours at room temperature to allow covalent attachment of the radio-labeled protein to the activated groups on the particles. Attachment of protein was quenched by the addition of bovine serum albumin (250 µl, 100 mg/ml) to each tube, followed by rotation of the capped tubes for an additional 20 hours at room temperature.

The total amount of radio-labeled protein bound to the particles was determined by measuring: a) the total counts per minute (CPM) in a sample of each suspension (125 µl in duplicate), and b) the cpm remaining in 125 µl of the supernatants following centrifugation of the remaining reaction mixture after removal of 250 µl aliquots to determine the fraction of the protein which was covalently bound to the particles. Each sample (250 µl total) was mixed with buffer (650 µl, 0.1 molar) and sodium dodecyl sulfate (100 µl of a 10% solution in deionized distilled water). The resulting mixtures were mixed by tumbling at 37°C for 16 hours on a rotating disc mounted at a 45° angle. This treatment removed adsorbed protein, but not covalently bound protein. The same procedure described above for determining the total amount of bound protein was used to determine the amount of protein covalently bound.

The total amount of ³H bovine gamma globulin bound to the particles, the amount covalently bound thereto, and the covalent:total bound protein ratio are shown in Table I below. These results show that the activating agents used in Examples 1 and 2 are generally as efficient for attachment as the activating agent used in the Comparison method. However, as pointed out above, the activating agents used in Examples 1 and 2 are readily available and the Comparison activating agent is not. Thus, the methods of this invention provide significant economic advantage over the Comparison method.

**TABLE I**

| Experiment | Polymer | Total % % Bound | Covalently Bound | Covalent/Total Ratio* |
|---|---|---|---|---|
| Example 1 | A | 97.5 | 95.8 | 98 |
| Example 2 | A | 96.2 | 91.4 | 95 |
| Comparison | A | 96.5 | 96.8 | 100 |
| Example 1 | B | 93.6 | 91.8 | 98 |
| Example 2 | B | 82.8 | 58.6 | 71 |
| Comparison | B | 88.7 | 87.7 | 99 |

| | | | | |
|---|---|---|---|---|
| * (x 100) | | | | |

### EXAMPLE 3 Preparation of Reagents Having Antibodies

This example demonstrates the practice of this invention to covalently attach antibodies specific to thyroxine to polymeric particles using an activating agent within the scope of this invention. The invention was also compared to a "Comparison" reagent prepared by a method outside the scope of this invention.

The procedure described in Examples 1 and 2 was used to covalently attach anti-thyroxine monoclonal antibodies (Beckman Instruments, Inc.) to Polymers A and B described above, except that an antibody solution (32.1 µl for Polymer A and 39.3 µl for Polymer B, 4.17 mg protein/ml) was used in place of the radio-labeled bovine gamma globulin. The reaction was quenched using bovine serum albumin, the suspensions were centrifuged, the supernatants decanted, and the particles were resuspended in phosphate buffered saline solution (1 ml, pH 7.4). This step was repeated three times, and during the last time, the solids were resuspended in the buffered solution (1.8 ml) and merthiolate preservative (0.02%) was added. The Comparison experiment was carried out similarly to the Comparison method described in Examples 1 and 2.

The relative amounts of active antibody in the resulting reagent dispersions were determined in an assay in which serial dilutions (from 1 x 10⁻⁷ to 1 x 10⁻¹⁰ molar theoretical antibody binding sites based on the mass of antibody bound to the particles) of the reagent suspensions were mixed with a fixed concentration (5 x 10⁻¹¹ molar) of horseradish peroxidase-labeled thyroxine. This labeled conjugate had been prepared using a procedure similar to that described by Kunst, Clin.Chem. 34(9), pages 1830-1833 (1988). The dilutions were incubated for about one hour with constant agitation at room temperature in phosphate buffered saline solution containing bovine serum albumin (1%) and 8-anilino-1-naphthalenesulfonic acid ammonium salt (8.7 x 10⁻⁴ molar). The amount of thyroxine label remaining in the suspension after centrifugation was determined, and the concentration of thyroxine binding sites required to bind 30% of the labeled thyroxine was calculated. The results are summarized in Table II below.

These data demonstrate that antibody activity was retained to a high degree with the practice of the present invention.

**TABLE II**

| Polymer | Theoretical Thyroxine Binding Sites Where 30% of Label is Bound (nmolar) | | |
|---|---|---|---|
| | Example 1 | Example 2 | Comparison |
| A | 19.5 | 10.0 | 14.4 |
| B | 26.4 | 23.4 | 17.3 |

### Example 4 Reagents with Antibodies Specific to Carbamazepine

This example illustrates the practice of this invention to prepare reagents useful in an immunoassay for carbamazepine. It is also a comparative example showing the practice of the present invention to the preparation of a a reagent using an activating agent outside the scope of this invention ("Comparison").

The particles for the reagents were prepared from poly[styrene-co-3-(p-vinylbenzylthio)propionic acid] (97.59:2.41 molar ratio, 1.2 µm average diameter) using a procedure like that described in Example 1 above. The final dispersions for the reaction comprised 0.25 mg antibody per 30 mg dry weight of particles in 2-(4-morpholino)ethane sulfonic acid buffer (0.1 molar, pH 5.5). Each activating agent was used in an amount of 30 µmole. The activating agent 2-(1H-benzotriazolyloxy)1,1,3,3-tetramethyluronium tetrafluoroborate was used in the practice of this invention in comparison to the use of 1-(1-pyrrolidinylcarbonyl)pyridinium chloride as a "Comparison" activating agent.

The dispersions were prepared by putting the polymer particle suspensions (30 mg dry weight) in large microfuge tubes in buffer (500 µl). The suspensions were then centrifuged for 10 minutes at 14,000 rpm and the supernatants discarded. Buffer (747 µl) and activating agent (267 µl of 112.5 mmolar stock solutions of each prepared in buffer) were then added to the tubes. The tubes were capped and rotated end-over-end at room temperature for 10 minutes. A solution of the antibody specific to carbamazepine (97 µl of a 2.59 mg/ml stock solution) was added to each tube. The tubes were then rotated again for four hours at room temperature. The reactions were quenched with bovine serum albumin (300 µl, 100 mg protein/ml) overnight at room temperature. Excess protein was removed as described in Example 3 above.

The relative amounts of active antibody in the resulting dispersions of reagents were determined in an assay similar to that described in Example 3 above, except that 8-anilino-1-naphthalenesulfonic acid was omitted from the assay. The concentration of carbamazepine binding sites required to bind 50% of the carbamazepine label was calculated. The results obtained were as follows:

| Theoretical Carbamazepine Binding Sites Where 50% of the Label is Bound (nmolar): | |
|---|---|
| Example 3 Assay | 0.51 |
| Comparison Assay | 0.59 |

This example demonstrates the effective attachment of antibody to polymeric particles with an activating agent which is readily available for use.

## Claims

1. A method for reacting a biologically active compound having a reactive amino or sulfhydryl group with a water-insoluble carboxylated substrate comprising:
A. contacting a water-insoluble substrate having reactive carboxy groups with a 2-(N-heterocyclyloxy)uronium salt represented by the structure: 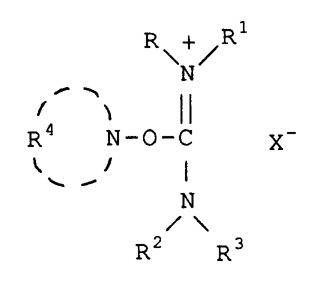 wherein R, R¹, R² and R³ are independently hydrogen, substituted or unsubstituted alkyl having 1 to 6 carbon atoms, substituted or unsubstituted alkenyl having 2 to 6 carbon atoms or alkynyl having 2 to 6 carbon atoms, or
R and R¹, when taken together, or R² and R³, when taken together, represent the carbon, sulfur, nitrogen and oxygen atoms necessary to complete a 5- to 7-membered substituted or unsubstituted heterocyclic ring with the nitrogen atom to which they are attached, or
R¹ and R³, when taken together, represent the carbon, sulfur, nitrogen and oxygen atoms necessary to complete a 5- to 7-membered substituted or unsubstituted heterocyclic ring with the uronium group to which they are attached,
R⁴ represents the carbon and heteroatoms which, when taken with the nitrogen atom attached to the oxy group, are sufficient to complete a 5- to 10-membered substituted or unsubstituted heterocyclic ring, and
X⁻ represents an acid anion,
to convert the reactive carboxy groups to activated groups in the substrate, and
B. contacting the substrate having activated groups with a biologically active compound having a reactive amino or sulfhydryl group which reacts with the activated groups to form a covalent linkage between the substrate and the biologically active compound.

2. The method as claimed in claim 1 wherein the carboxylated substrate is a water-insoluble carboxylated particle.

3. The method as claimed in any of claims 1 and 2 wherein the biologically active compound is a polypeptide, protein, carbohydrate derivative, hapten, hormone, lectin, vitamin, nucleic acid or an oligonucleotide.

4. The method as claimed in any of claims 1 to 3 wherein the substrate or the biologically active compound used in step B has a detectable tracer associated therewith.

5. The method as claimed in any of claims 1 to 4 wherein the uronium salt is used in step A in an amount of from 1:1 to 200:1 molar ratio of uronium salt to the total measured carboxylic acid level in the substrate, the biologically active compound is used in step B in an amount of from 1:1000 to 2:1 weight ratio of compound to substrate, and step A is carried out at a temperature of from 10 to 60°C for up to 20 minutes, and step B is carried out at a temperature of from 10 to 50°C for up to 24 hours.

6. A kit comprising a water-insoluble substrate having reactive carboxy groups,
the kit characterized as further comprising, individually packaged, a 2-(N-heterocyclyloxy)uronium salt represented by the structure: 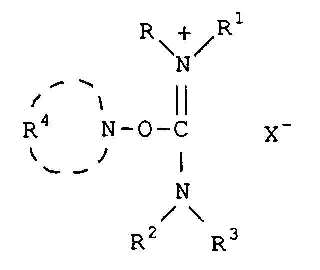 wherein R, R¹, R² and R³ are independently hydrogen, substituted or unsubstituted alkyl having 1 to 6 carbon atoms, substituted or unsubstituted alkenyl having 2 to 6 carbon atoms, or alkynyl having 2 to 6 carbon atoms, or
R and R¹, when taken together, or R² and R³, when taken together, represent the carbon, sulfur, nitrogen and oxygen atoms necessary to complete a 5- to 7-membered substituted or unsubstituted heterocyclic ring with the nitrogen atom to which they are attached, or
R¹ and R³, when taken together, represent the carbon, sulfur, nitrogen and oxygen atoms necessary to complete a 5- to 7-membered substituted or unsubstituted heterocyclic ring with the uronium group to which they are attached,
R⁴ represents the carbon and heteroatoms which, when taken with the nitrogen atom attached to the oxy group, are sufficient to complete a 5- to 10-membered substituted or unsubstituted heterocyclic ring, and
X⁻ represents an acid anion.

7. The kit as claimed in claim 6 further comprising a separately packaged biologically active compound having a reactive amino or sulfhydryl group.

8. The method as claimed in any of claims 1 to 5 or the kit as claimed in any of claims 6 or 7 wherein each of R, R¹, R² and R³ is hydrogen, alkyl of 1 to 3 carbon atoms or alkenyl having 2 to 4 carbon atoms, R⁴ represents the carbon and heteroatoms which, when taken with the nitrogen atom attached to the oxy group, are sufficient to complete a 5- to 9-membered heterocyclic ring, the heterocyclic ring being unsubstituted or substituted with up to two of the same or different substituents which are oxo, lower alkoxy, halo, or lower alkyl, and X⁻ is halide, triflate, sulfate, tetrafluoroborate, hexafluorophosphate, hydroxide, methosulfate, perchlorate, nitrate or p-toluenesulfonate.

9. The method as claimed in any of claims 1 to 5 or the kit as claimed in any of claims 6 or 7 wherein the uronium salt is 2-(1H-benzotriazol-1-yloxy)-1,1,3,3-tetra-methyluronium tetrafluoroborate or 2-(succinimidoxy)-1,1,3,3-tetramethyluronium tetrafluoroborate.
